(19) Europäisches Patentamt
European Patent Office
Office européen des brevets

(11) Veröffentlichungsnummer: **0 142 860**
**B1**

(12) **EUROPÄISCHE PATENTSCHRIFT**

(45) Veröffentlichungstag der Patentschrift: **29.08.90**

(21) Anmeldenummer: **84114038.7**

(22) Anmeldetag: **20.11.84**

(51) Int. Cl.$^5$: **C 07 K 7/10**, A 61 K 37/02, C 12 Q 1/37, C 07 K 1/12

(54) **Desulfatohirudine, Verfahren zu ihrer Herstellung und pharmazeutische Mittel.**

(30) Priorität: **22.11.83 DE 3342139**

(43) Veröffentlichungstag der Anmeldung:
**29.05.85 Patentblatt 85/22**

(45) Bekanntmachung des Hinweises auf die Patenterteilung:
**29.08.90 Patentblatt 90/35**

(84) Benannte Vertragsstaaten:
**AT BE CH DE FR GB IT LI LU NL SE**

(56) Entgegenhaltungen:

**CHEMICAL ABSTRACTS, Band 85, 1976, Seite 234, Zusammenfassung Nr. 105932p, Columbus, Ohio, US; T.E. PETERSEN et al.: "Primary structure of hirudin, a thrombin-specific inhibitor"**
**CHEMICAL ABSTRACTS, Band 100, 1984, Seite 227, Zusammenfassung Nr. 81858u, Columbus, Ohio, US; J.Y. CHANG: "The functional domain of hirudin, a thrombin-specific inhibitor"**

(73) Patentinhaber: **CIBA-GEIGY AG**
**Klybeckstrasse 141**
**CH-4002 Basel (CH)**
(73) Patentinhaber: **UCP GEN-PHARMA AG**
**Solothurnstrasse 24**
**CH-3422 Kirchberg (CH)**

(72) Erfinder: **Seemüller, Ursula, Dr.**
**Destouches-Strasse 60**
**D-8000 München 40 (DE)**
Erfinder: **Dodt, Johannes, Dipl.-Chem.**
**Aberlestrasse 4**
**D-8000 München 70 (DE)**
Erfinder: **Fritz, Hans, Prof. Dr.**
**Neulinger Strasse 15**
**D-8011 Hohenbrunn (DE)**
Erfinder: **Fink, Ernst, Prof. Dr.Dr.**
**Stellhorner Strasse 1**
**D-2901 Westerstede-Giesselhorst (DE)**

(74) Vertreter: **Kinzebach, Werner, Dr.**
**Patentanwälte Reitstötter, Kinzebach und Partner Sternwartstrasse 4 Postfach 86 06 49**
**D-8000 München 86 (DE)**

**EP 0 142 860 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:

CHEMICAL ABSTRACTS, Band 100, 1984, Seite 227, Zusammenfassung Nr. 152937g, Columbus, Ohio, US; J. DODT et al.: "The complete amino acid sequence of hirudin, a thrombin specific inhibitor. Application of color carboxymethylation"
Seemüller et al.- "Proteinase Inhibitors", Elsevica Science Publisher BV, 1986
Stone et al.: Biochemistry 25, 4622 (1986)

Die Akte enthält technische Angaben, die nach dem Eingang der Anmeldung eingereicht wurden und die nicht in dieser Patentschrift enthalten sind.

## Beschreibung

Die Erfindung betrifft neue, von Hirudin abgeleitete, biologisch aktive Polypeptide sowie Verfahren zu deren Herstellung, diese enthaltende pharmazeutische Zusammensetzungen und ihre Verwendung, insbesondere zur Hemmung der Blutgerinnung.

Hirudin, von dem sich die erfindungsgemäßen Verbindungen strukturell ableiten, ist ein polypeptidischer Naturstoff, der im Organismus von medizinischen Blutegeln (Hirudo medicanalis) produziert wird und der das vom Egel aufgenommene Blut in ungeronnenem Zustand hält. Seine Isolierung, Reinigung, chemische Zusammensetzung sowie breite biologische und medizinische Anwendung als Antikoagulans sind bekannt und in Übersichtsartikeln, z.B.P. walsmann und F. Markwardt, Pharmazie *36*, 653—660 (1981), zusammengefaßt und eingehend diskutiert. In jüngster Zeit wurde schließlich die vollständige Aminosäure-Sequenz von Hirudin aufgeklärt und dadurch auch die erste theoretische Grundlage für Versuche zu dessen synthetischer Herstellung geschaffen. Die Primärstruktur von Hirudin entspricht der Formel:

```
                                              10
H-Val-Val-Tyr-Thr-Asp-Cys-Thr-Glu-Ser-Gly-
                                              20
 -Gln-Asn-Leu-Cys-Leu-Cys-Glu-Gly-Ser-Asn-
                                              30
 -Val-Cys-Gly-Gln-Gly-Asn-Lys-Cys-Ile-Leu-       (A)
                                              40
 -Gly-Ser-Asp-Gly-Glu-Lys-Asn-Gln-Cys-Val-
                                              50
 -Thr-Gly-Glu-Gly-Thr-Pro-Lys-Pro-Gln-Ser-
                                              60
 -His-Asn-Asp-Gly-Asp-Phe-Glu-Glu-Ile-Pro-
              SO3H
              |
 -Glu-Glu-Tyr-Leu-Gln-OH
```

(Welche spezifischen Cysteinreste sich miteinander durch Disulfid-Brücken paaren, bleibt noch .zu ermitteln; für die Syntheseplannung ist diese Strukturdetail von geringer Bedeutung.) Die Struktur zeichnet sich aus durch eine charakteristische Anhäufung von hydrophoben Aminosäuren gegen das Aminoende und von polaren Aminosäuren gegen das Carboxylende des Peptids sowie, als ein besonderes Merkmal, durch die stark saure Sulfatmonoestergruppe am phenolischen Hydroxyl des Tyrosin-Restes in Stellung 63 entsprechend der Teilformel

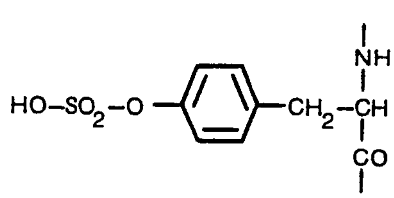 bzw. [Tyr $(SO_3H)^{63}$]

Über die biologische Funktion des Sulfatrestes in Proteinen allgemeinen und in diesem Wirkstoff speziell gab es bislang keine klaren Vorstellungen. Folgende Hypothesen wurden diskutiert:

(1) eine Bedeutung für die biologische Aktivität des Proteins;

(2) eine Beteiligung an regulatorischen Zellprozessen (analog, wie es für die reversible Phosphorylierung bekannt ist);

(3) eine Stimulierung der Sekretion, d.h. die Sulfatierung dient als Markierung für die Erkennung als sekretorisches Protein: alle bislang entdeckten sulfatierten Proteine sind sekretorische oder transmembranale Proteine. Jedenfalls ist der Sulfatrest eines der markantesten Strukturmerkmale von Hirudin.

Biologisch gehört Hirudin mit dem $K_i$-Wert von etwa $6,10^{-11}$M zu den wirksamsten Thrombin-Inhibitoren; dabei wirkt es gegen Thrombin ganz spezifisch und inhibiert keine anderen Proteinasen der Blutgerinnungskaskade. Im Gegensatz zu Heparin übt Hirudin seinen inhibierenden Einfluß auf Thrombin direkt aus und wirkt nicht, wie jenes, durch Antithrombin III. Der einzige pharmakologisch faßbare Effekt von gereinigtem Hirudin ist die Hemmung der Blutkoagulation bzw. die Prophylaxe von Thrombose. bei intravenöser Verabreichung an Hunde war sogar bei ungewöhnlich hohen Dosen kein Einfluß auf Herzschlagfrequenz, Atmung, Blutdruck, Thrombocytenzahl, Fibrinogen und Hämoglobin zu beobachten. Bei Versuchen an der Ratte, am Schwein und am Hund erwies sich Hirudin als wirksam bei experimeteller Thrombosis (hervorgerufen entweder durch Stasis oder durch Injektion von Thrombin), beim Endotoxin-Schock sowie bei DIC (disseminierter intravasaler Gerinnung). Bei direkten Vergleichsversuchen mit Heparin, wo immer sie durchgeführt wurden, zeigte sich Hirudin diesem überlegen.

Obwohl seit langem bekannt, erlangte Hirudin bis heute nicht die breite therapeutische Anwendung, die man aufgrund seiner ausgezeichneten biologischen Eigenschaften mit Recht erwarten durfte. Seiner allgemeinen Verbreitung in der Medizin steht nämlich als ein schwerwiegender Nachteil seine äußerst beschränkte Zugänglichkeit im Wege. Bis jetzt waren Hirudin-Präparate ausschließlich aus teuerm und schwer zugänglichem Naturmaterial — den medizinischen Blutgeln — durch aufwendige Isolier- und Reinigungsverfahren erhältlich. Die verhältnismäßig lange Sequenz von 65 Aminosäuren beitet einer synthetischen Annäherung durch die klassische Peptid-Synthese kaum Hoffnung auf einen praktischen Erfolg an. Ebensowenig aussichtsvoll schien aber auch der alternative, biosynthetische Zugang zu sein, der durch die Synthese eines geeigneten Polynucleotids und dessen Eingliederung in den genetischen Code eines Produktionsmikroorganismus gemäß den allgemeinen Arbeitsmethoden der Gen-Manipulation zustande kommen könnte. Es war zu erwarten, daß einer direkten Biosynthese die notwendige Einführung des O-sulfonierten Tyrosin-Restes fast unüberwindbare Schwierigkeiten bereiten würde.

Überraschenderweise wurde nun festgestellt, daß entgegen den obigen theoretischen Vortstellungen diei günstigen biologischen Eigenschaften von Hirudin auch dann erhaltenbleiben, wenn die charakteristische Sulfatmonoester-Gruppe vom phenolischen Hydroxyl des [Tyr$^{63}$]-Restes entfernt wird. Die resultierenden Abbauprodukte sind die Desulfatohirudine der Formeln I und II

```
                                           10
H-Val-Val-Tyr-Thr-Asp-Cys-Thr-Glu-Ser-Gly-
                                           20
 -Gln-Asn-Leu-Cys-Leu-Cys-Glu-Gly-Ser-Asn-
                                           30
 -Val-Cys-Gly-Gln-Gly-Asn-Lys-Cys-Ile-Leu-   (I)
                                           40
 -Gly-Ser-Asp-Gly-Glu-Lys-Asn-Gln-Cys-Val-
                                           50
 -Thr-Gly-Glu-Gly-Thr-Pro-Lys-Pro-Gln-Ser-
                                           60
 -His-Asn-Asp-Gly-Asp-Phe-Glu-Glu-Ile-Pro-
 -Glu-Glu-Tyr-Leu-Gln-OH
```

und der Formel II

```
                                                10
H-Val-Val-Tyr-Thr-Asp-Cys-Thr-Glu-Ser-Gly-
                                                20
-Gln-Asn-Leu-Cys-Leu-Cys-Glu-Gly-Ser-Asn-
                                                30
-Val-Cys-Gly-Gln-Gly-Asn-Lys-Cys-Ile-Leu-   (II)
                                                40
-Gly-Ser-Asp-Gly-Glu-Lys-Asn-Gln-Cys-Val-
                                                50
-Thr-Gly-Glu-Gly-Thr-Pro-Lys-Pro-Gln-Ser-
                                                60
-His-Asn-Asp-Gly-Asp-Phe-Glu-Glu-Ile-Pro-
-Glu-Glu-Tyr-OH
```

Die Desulfatohirudine sind bis auf die Abwesenheit der Sulfatestergruppe am $Tyr^{63}$ durch alle übrigen Strukturmerkmale des Hirudins charakterisiert, wobei beim Desulfatohirudin der Formel I, welches das eigentliche Desulfatohirudin im engeren Sinne des Wortes darstellt, die ungekürzte Aminosäuresequenz vorliegt, während im analogen, wirkungsmäßig gleichwertigen Hexacontatripeptid, bezeichnet als Desulfatohirudin der Formel II, zusätzlich noch die beiden C-terminalen Aminosäuren Leu und Gln fehlen. Überraschenderweise sind diese Verbindungen jedoch Hirudin in biologischer Hinsicht, insbesondere in ihrer antikoagulativen Wirkung qualitativ und quantitativ zumindest ebenbürtig.

Diese Feststellung ist in bezug auf die Möglichkeiten einer konventionellen biotechnologischen Synthese dieses Peptids von großer Bedeutung: während die Anwesenheit der ungewöhnlichen Hydroxysulfonylgruppe bei Hirudin dessen direkte Biosynthese praktisch ausschließt, sind durch ihr Fehlen bei Desulfatohirudin wesentlich günstigere strukturelle Voraussetzungen für eine erfolgreiche biotechnische Synthese vorhanden. Bei Gleichheit der biologischen Wirksamkeit ist somit Desulfatohirudin aufgrund der wesentlich besseren Zugänglichkeit auf biotechnologischem Wege dem Hirudin in technisch-ökonomischer Hinsicht klar überlegen.

Erfindungsgemäß sind die Desulfatohirudine der Formel I und II in an sich bekannter Weise erhältlich. So kann man sie beispielsweise herstellen, indem man in Hexacontapentapeptid Hirudin der oben angegebenen Formel (A) die als Schwefelsäure-monoester vorhandene phenolische Hydroxylgruppe des Tyrosin-Restes in Stellung 63 freisetzt.

Die Freisetzung dieser Gruppe entsprechend dem Schema

$$HO—SO_2—O—Pept \rightarrow HO—Pept$$

(worin Pept den restlichen Teil von Hirudin darstellt) kann in an sich bekannter Weise, z.B. durch Hydrolyse, erfolgen, wobei sowohl chemische als auch biologische Methoden zu diesem Zweck anwendbar sind.

Bei einer chemischen Freisetzung wird vorzugsweise unter den allgemeinen Bedingungen der säurekatalysierten Hydrolyse gearbeitet, wie unter des Einwirkung einer verdünnten, z.B. einer etwa 2- bis etwa 4N wäßrigen Salzsäurelösung, vorteilhaft in Trifluoressigsäure als Reaktionsmedium, oder mit wasserhaltiger Trifluoressigsäure allein als Reaktions- und Lösungsmittel. Um die Gefahr der hydrolytischen Spaltung von Peptidbindungen auf einem Minimum zu halten, ist es empfehlenswert, unter milden Reaktionsbedingungen, z.B. bei Temperaturen, welche Zimmertemperatur nicht übersteigen, zu arbeiten und den Fortschritt der Hydrolyse analytisch, z.B. durch Dünnschichtchromatographie, zu verfolgen.

Vornehmlich erfolgt jedoch die Hydrolyse auf biologischem Wege, insbesondere durch die Anwendung von spezifischen Enzymen, Arylsulfatasen, welche die phenolischen Sulfatestergruppen zu freien phenolischen Gruppen unter milden Bedingungen spalten. Die biologische Freisetzung der sulfatierten Hydroxylgruppe kann mit Hilfe eines geeigneten Enzympräparats mit angereichertem Wirkstoff oder eines isolierten Enzyms erfolgen, oder aber man kann eine geeignetes Enzymsystem in situ einsetzen, d.h. wie es in einem lebenden oder abgetöteten biologischen Material, z.B. einem wachsenden oder ruhenden Mikroorganismus, einer Zellkultur, einem Zellhomogenat oder einem Autolysat, unmittelbar vorliegt. Einer der größten Vorteile der bioligischen Hydrolyse ist ihre hohe Selektivität, die nur die

gewünschte Spaltung der Monosulfatester-Bindung bewirkt, ohne die übrigen funktionellen Gruppen, vor allem die peptidischen Bindungen, im empfindlichen Ausgangsstoff anzugreifen. Vornehmlich werden die erfindungsgemäßen Verbindungen hergestellt, indem man Hirudin in einer wäßrigen, vorzugsweise gepufferten Lösung oder Suspension mit einem individuellen Arylsulfatase-Präparat, z.B. der Arylsulfatase aus Helix pomatia, bei einer für enzymatische Prozesse gebräuchlichen Temperatur, wie etwa 20 bis 45 und vorzugsweise 25 bis 30°C, behandelt. Vorzugsweise wird bei einer schwach sauren Reaktion, d.h. bei einem pH von etwa 4 bis etwa 7, insbesondere von etwa 5 bis etwa 6, gearbeitet, welcher mit einem Puffer, wie einer etwa 0,03- bis etwa 0,3-molaren Lösung eines Salzes einer organischen Carbonsäure mit einem Alkalimetall oder mit einer organischen Base, z.B. mit Natriumacetat bzw. insbesondere Pyridinacetat (vom pH etwa 5,4), eingestellt wird. Das Verhältnis des eingesetzten Enzyms zum Substrat (Hirudin) richtet sich im allgemeinen nach der Aktivität des jeweiligen Präparats und beträgt üblicherweise von etwa 1:2 bis etwa 1:100, insbesondere von etwa 1:5 bis etwa 1:20; es ist vorteilhaft, Enzympräparate von höchstmöglichem Reinheitsgrad und Aktivität zur verwenden. Da die Arylsulfatasen nicht nur die Abspaltung, sondern auch die Einführung der Sulfatgruppe katalysieren und das Einstellen eines Gleichgewichts der Ausgangs- und Endstoffe bewirken, ist es vorteilhaft, für jedes Enzympräparat durch Vorversuche die optimale Konzentration, das Mengenverhältung zum Substrat und den Zeitverlauf der Desulfatierung festzustellen. In der Regel ist aber die Reaktion schon nach wenigen Minuten beendet; die Qualität des Reaktionsproduktes wird auch bei längerem (bis etwa 4stündigen) Kontakt mit aktivem Enzym (z.B. beim Stehen des Reaktionsgemisches) nicht beeinträchtigt.

Der Verlauf der enzymatischen Desulfatisierung kann bioanalytisch an entnommenen Proben vorfolgt werden: praktisch geht man z.B. so vor, daß man durch kurzes (etwa 3minütiges) Erhitzen der Probe auf etwa 100°C die Enzymaktivität zerstört und das Substrat mit einer Carboxylpeptidase Y behandelt. (Die Carboxypeptidase Y baut die Peptidkette von der Carboxyseite her ab, indem die Aminosäuren nacheinander durch Spalten der jeweiligen Amidbindungen abgespalten werden). Üblicherweise ist der Abbau der Peptidkette nach etwa 15 min so fortgeschritten, daß die betreffende, sulfattisierte und/oder freie 63-ständige Aminosäure ($Tyr^{63}$) vollständig abgespalten und somit der Bestimmung in einem konventionellen Aminosäure-Analysator zugänglich ist.

Das Desulfatohirudin der Formel II entsteht durch Abspaltung der beiden C-terminalen Aminosäurebausteine Leu und Gln im Laufe der Hydrolyse von Hirudin. Die Auftrennung des dabei anfallenden Gemisches kann beispielsweise durch präparative HPLC-Chromatographie erfolgen. Das Desulfatohirudin der Formel II besitzt die gleichen biologischen Eigenschaften wie das Desulfatohirudin der Formel I.

Die erfindungsgemäßen Desulfatohirudine können nicht nur in freier Form, sondern auch in Form ihrer Salze vorliegen. Da sie in mehreren Aminosäureresten freie Aminogruppen bzw. Amidinogruppen enthalten, können die erfindungsgemäßen Verbindungen in Form von Säureadditionssalzen vorliegen. Als Säureadditionssalze kommen insbesondere physiologisch verträgliche Salze mit üblichen, therapeutisch anwendbaren Säuren in Betracht; als anorganische Säuren sind die Halogenwasserstoffsäuren (wie die Chlorwasserstoffsäure), aber auch Schwefelsäure und Phosphor- bzw. Pyrophosphorsäure zu nennen; als organische Säuren sind in erster Linie Arensulfonsäuren (wie die Benzol- oder p-Toluolsulfonsäure oder Niederalkansulfonsäuren, wie Methansulfonsäure) sowie Carbonsäuren, wie Essigsäure, Milchsäure, Palmitin- und Stearinsäure, Äpefelsäure, Weinsäure, Ascorbinsäure und Citronensäure, geeignet. Da die Desulfatohirudine aber auch in mehreren Aminosäureresten freie Carboxylgruppe enthalten, die dem ganzen Peptid sauren Charakter verleihen, können sie auch als Salz vorliegen, z.B. Natrium-, Kalium-, Calcium- oder Magnesiumsalz, oder auch als Ammoniumsalz, abgeleitet von Ammoniak oder einer physiologisch verträglichen, organischen, stickstoffhaltigen Base. Da sie aber zugleich freie Carboxylgruppen und freie Amino (und Amidino)-Gruppen enthalten, können sie auch als inneres Salz vorliegen.

Je nach Arbeitsweise erhält man die erfindungsgemäßen Verbindungen in freier Form oder in Form von Säureadditionssalzen, inneren Salzen oder Salzen mit Basen. Aus den Säureadditionssalzen kann in an sich bekannter Weise die freie Verbindung gewonnen werden. Von letzterer wiederum lassen sich durch Umsetzem mit Säuren, z.B. mit solchen, die die obengenannten Salze bilden, und Eindampfen oder Lyophilisieren therapeutisch anwendbare Säureadditionssalze gewinnen. Die inneren Salze können durch Einstellen des pH auf einen geeigneten Neutralpunkt gewonnen werden.

Die Erfindung betrifft ebenfalls pharmazeutische Zusammensetzungen, welche wenigstens eine der erfindungsgemäßen Verbindungen oder deren pharmazeutisch verwendbare Salze, gegebenenfalls zusammen mit einem pharmazeutischen Träger und/oder Hilfsstoffen, enthalten.

Diese Zusammensetzungen können insbesondere bei den oben angegebenen Indikationen Verwendung finden, wenn sie z.B. parenteral (wie intravenös, intracutan, intramuskulär oder subcutan), oral oder topisch verabreicht werden. Die Dosierung hängt in erster Linie von der spezifischen Verabreichungsform und vom Zweck der Therapie bzw. Prophylaxe ab. Die Größe der Einzeldosen sowie das Verabreichungsschema kann am besten anhand einer individuellen Beurteilung des jeweiligen Krankheitsfalles bestimmt werden; die dazu erforderlichen Methoden zur Bestimmung von relevanten Blutfaktoren sind dem Fachmann geläufig. Im Normalfall liegt bei einer Injektion die therapeutisch wirksame Menge der erfindungsgemäßen Verbindungen im Dosisbereich von etwa 0,005 bis etwa 0,1 mg/kg Körpergewicht. Bevorzugt wird der Bereich von etwa 0,01 bis etwa 0,05 mg/kg Körpergewicht. Die

Verabreichung erfolgt durch intravenöse, intramuskuläre oder subcutane Injektion. Dementsprechend enthalten pharmazeutische Präparate zur parenteralen Verabreichung in Einzeldosis-Form in Abhängigkeit von der Applikationsart pro Dosis etwa 0,4 bis etwa 7,5 mg der erfindungsgemäßen Verbindung. Neben dem Wirkstoff enthalten diese pharmazeutischen Zusammensetzungen üblicherweise noch einen Puffer, z.B. einen Phosphatpuffer, der den pH-Wert zwischen etwa 3,5 und 7 halten soll, und ferner Natriumchlorid, Mannit oder Sorbit zur Einstellung der Isotonie. Sie können in gefreiergetrockneter oder gelöster Form vorliegen, wobei Lösungen mit Vorteil ein antibakteriell wirkendes Konservierungsmittel, z.B. 0,2 bis 0,3% 4-Hydroxybenzosäure-methylester oder -ethylester, enthalten können.

Ein Präparat für die topische Anwendung kann als wäßrige Lösung, Lotion oder Gelee, ölige Lösung oder Suspension, oder fetthaltige oder insbesondere Emulsions-Salbe vorliegen. Ein Präparat in Form einer wäßrigen Lösung erhält man beispielsweise dadurch, daß man die erfindungsgemäßen Wirkstoffe oder ein therapeutisch anwendbares Salz davon in einer wäßrigen Pufferlösung von pH 4 bis 6,5 löst und gewünschtenfalls einen weiteren Wirkstoff, z.B. ein Antiinflammatorikum, und/oder eine polymeres Haftmittel, z.B. Polyvinylpyrrolidon, und/oder ein Konservierungsmittel zufügt. Die Konzentration des Wirkstoffs beträgt etwa 0,08 bis etwa 1,5 mg, vorzugsweise 0,25 bis 1,0 mg, in etwa 10 ml einer Lösung bzw. 10 g eines Gelees.

Eine ölige Applikationsform für die topische Verabreichung erhält man beispielsweise durch Suspendieren der erfindungsgemäßen Wirkstoffe oder eines therapeutisch anwendbaren Salzes davon in einem Öl, gegebenenfalls unter Zusatz von Quellmitteln, wie Aluminium-stearat, und/oder grenzflächenaktiven Mitteln (Tensiden), deren HLB-Wert ("hydrophilic-lipophilic-balance") unter 10 liegt, wie Fettsäuremonoester mehrwertiger Alkohole, z.B. Glycerinmonostearat, Sorbitanmonolaurat, Sorbitanmonostearat oder Sorbitanmooleat. Eine fetthaltige Salbe erhält man z.B. durch Suspendieren der erfindungsgemäßen Wirkstoffe oder deren Salze in einer streichbaren Fettgrundlage, gegebenenfalls unter Zusatz eines Tensids vom HLB-Wert unter 10. Eine Emulsionssalbe erhält man durch Verreiben einer wäßrigen Lösung der erfindungsgemäßen Wirkstoffe oder deren Salze in einer weichen, streichbaren Fettunterlage unter Zusatz eines Tensids, dessen HLB-Wert unter 10 liegt. Alle diese topischen Applikationsformen können auch Konservierungsmittel enthalten. Die Konzentration des Wirkstoffes beträgt etwa 0,08 bis etwa 1,5 mg, vorzugsweise 0,25 bis 1,0 mg, in etwa 10 g der Grundmasse.

Neben den oben beschriebenen und ihnen analogen pharmazeutischen Zusammensetzungen, welche für einen direkten medizinischen Einsatz am Körper des Menschen oder eines Säugetieres bestimmt sind, betrifft die vorliegende Erfindung auch pharmazeutische Zusammensetzungen und Präparate zur medizinischen Anwendung außerhalb des lebenden Körpers des Menschen oder der Säugetiere. Solche Zusammensetzungen und Präparate verwendet man in erster Linie als gerinnungshemmenden Zusatz zu Blut, welches außerhalb des Körpers einer Zirkulation oder Behandlung (z.B. Dialyse in künstlichen Nieren), Konservierung oder Modifizierung (z.B. Haemoseparation) unterzogen wird. In ihrer Zusammensetzung sind derartige Präparate, wie Vorratslösungen oder auch Zubereitungen in Einzeldosis-Form, den oben beschriebenen Injektionspräparaten ähnlich; zweckmäßigerweise wird aber die Wirkstoffmenge bzw. -konzentration auf das Volumen des zu behandelnden Blutes oder, noch exakter, auf dessen Thrombingehalt bezogen. Dabei ist zu beachten, daß die erfindungsgemäßen Wirkstoffe (in freier Form)

(a) eine etwa 5fache Gewichtsmenge Thrombin völlig deskativieren;

(b) auch in größeren Mengen physiologisch harmlos sind; und

(c) von zirkulierenden Blut auch in hohen Konzentrationen sehr schnell ausgeschieden werden, so daß keine Gefahr einer Überdosierung, auch z.B. bei Transfusionen, besteht. Je nach dem spezifischen Zweck beträgt die geeignete Dosis etwa 0,01 bis etwa 1,0 mg Wirkstoff/1 Blut, wobei die obere Grenze ohne Gefahr noch weit überschritten werden darf.

Zum Gegenstand der vorliegenden Erfindung gehört auch die bioanalytische Anwendung der erfindungsgemäßen Verbindungen und ihrer Salze zur Bestimmung von Thrombin, sowie die diesem Zweck dienenden, die erfindungsgemäßen Wirkstoff enthaltenden Präparate, z.B. Feststoffgemische und vor allem Lösungen, insbesondere wäßrige Lösungen; diese können neben einer genauen Menge bzw. Konzentration der erfindungsgemäßen Wirkstoffe (auch in Form eines Salzes) zweckmäßig noch inerte Hilfsstoffe enthalten, z.B. die oben bei Injektionspräparaten erwähnten, welche beispielsweise eine stabilisierende und/oder konservierende Aufgabe haben. Diese Präparate werden bei Bioanalysen in analoger, bekannter Weise wie die Hirudin-Präparate, beispielsweise zur Bestimmung von Thrombin, verwendet.

In der vorliegenden Beschreibung und in den Ansprüchen werden die Kurzbezeichnungen für Aminosäuren und ihre Reste im Einklang mit den allgemein angenommenen nomenklatorischen Regeln angewendet und beziehen sich auf α-Aminosäuren und deren Reste der in der Natur vorkommenden L-Reihe.

Die nachfolgenden Beispiele dienen zur Erläuterung der Erfindung.

### Beispiel 1

Material: Hirudin, Aktivität 630 IE/mg

Die biologische Aktivität wird über die Hemmwirkung gegenüber Thrombin bestimmt, dessen enzymatische Aktivität seinerseits wiederum gegenüber dem chromogen Substrat Chromozym TH (ein Produkt der Fa.Boehringer, Mannheim, BRD, für Thrombin- bzw. Hirudin-Bestimmung) gemäß bekannter,

mit dem Testpräparat gelieferter Vorschrift ermittelt wird.

Arylsulfatase (ARS) aus Helix pomatia (ein Produkt der Fa. Boehringer, Mannheim, BRD), 5 E/mg.

Die enzymatische Aktivität wird nach der bekannten Methode von Leon et al., Biochem. J. *75*, 612—617, mittels des chromogenen Substrats p-Nitrophenolsulfat (1,8 mMol/l im Ansatz) bestimmt.

Desulfatierung

(1) Vorversuch (Zur Ermittlung der optimalen ARS-Konzentration)

(a) Folgende Vorratslösungen werden zubereitet:

(A) Hirudin-Lösung von Konzentration 2 mg/ml, erhalten durch Lösen von Hirudin in Lösung (C).

(B) Arylsulfatase-Lösung von Konzentration 1,25 mg/ml: durch Vermischen von 25 Teilen der käuflichen Suspension mit 100 Teilen der Lösung (C).

(C) Pufferlösung: 0,1 M wäßrige Pyridinacetat-Lösung, pH 5,4.

(b) Prozedur:

Durch Vermischen der folgenden Komponenten wird eine Serie von Proben erhalten: jede Probe enthält 15 µl der Lösung A (entsprechend 30 µg Hirudin) und 10 µl der Lösung B (entsprechend 12,5 µg Arylsulfatase) oder einer Lösung, in welcher durch Verdünnen der Lösung B mit dem Puffer C die Konzentration des Enzyms auf ½, ¼, ⅛, 1/16 bzw. 1/32 der ursprünglichen gebracht wird. Die Proben von je 25 µl werden 60 min bei 25°C inkubiert, danach 3 min auf 100°C zwecks Denaturierung der Sulfatase erhitzt, rasch abgekühlt und auf Gehalt an freiem und sulfatiertem Tyrosin (gemäß der unten beschriebenen Methode) analysiert.

(2) Präparatives Verfahren

man vermischt 15 Vol-Teile der Lösung A und 10 Vol-Teile einer verdünnten Lösung B, deren jeweilige optimale, möglichst niedrige Konzentration im Vorversuch ermittelt wurde und durch Verdünnen der Vorratslösung B mit der Pufferlösung C eingestellt wird. Das Gemisch wird bei 25°C etwa 30 bis 60 min inkubiert, kurzzeitig (z.B. unter Bedingungen der "flash sterilization") auf 100°C erhitzt und sofort abgekühlt, um das Desulfierungsenzym zu denaturieren. Das Reaktionsgemisch wird, gegebenenfalls nach Einengen im Vakuum bei oder unterhalb Raumtemperatur, an einer Säule von Sephadex (R) G50 oder G75, CM-Sephaxex(R), Wofatit(R)CP, Amberlite(R)IRC oder einem anderen aquivalenten Kationenaustauscher getrennt. Nach Bedarf wird diese Trennung wiederholt, bis Desulfatohirudin gewünschter Reinheit (ermittelt z.B. durch den Hemmtest mit Thrombin und/oder die Aminosäureanalyse, siehe unten) resultiert. Das Produkt in fester Form wird durch Lyophilisierung der entsprechenden Lösungen (Eluate) erhalten. Reines Produkt soll nach der Aminosäure-Analyse (nach C-terminaler Proteolyse frei von Tyrosin-O-sulfat sein und im Hemmaktivitäts-Test gegenüber Thrombin (z.B. mittels Chromozym TH — siehe oben) die volle Aktivität des Hirudins aufweisen.

Analytische Kontrolle der Dersulfatisierung

erfolgt dadurch, daß am Hirudin (als Ausgangsstoff), Proben aus dem Desulfatierungsverfahren sowie Desulfatohirudin der Formel I (als Endstoff) vom Carboxylende her mit Carboxypeptidase Y stufenweise proteolytisch abbaut und die abgespaltenen Aminosäure-Reste mit Hilfe eines konventionellen Aminosäure-Analysators quantitativ bestimmt.

(a) Folgende Vorratslösungen werden zubereitet:

(Aa) Hirudin-Lösung der Konzentration 0,806 mg/ml wird durch Lösen von 0,250 Gew. Teilen Hirudin in 310 Vol-Teilen Pufferlösung Ca (siehe unten) erhalten.

(Ba) CPY-Lösung der Konzentration 2 mg/ml wird durch Lösen von 2 Gew. Teilen Carboxypeptidase Y (CPY) in 1000 Vol-Teilen Pufferlösung Ca erhalten.

(Ca) Pufferlösung: 0,1 M wäßrige Pyridinacetat-Lösung, pH 5,4.

(b) Prozedur:

275 µl Lösung As, entsprechend 222 µg Hirudin versetzt man mit 8 µl Lösung Ba, entsprechend 16 µg CPY, d.h. in einem Verhältnis zu Hirudin von 1:4 (Gew./Gew.) bzw. 1:125 (Mol/Mol), und inkubiert 30 min bei 25°C. Dem Gemisch wird eine Probe von 30 µl entnommen, mit 5 µl Trifluoressigsäure versetzt und zentrifugiert, um den CPY-Niederschlag zu entfernen; die überstehende Lösung wird eingetrocknet und die im Rückstand verbleibenden Aminosäuren werden in eine für die Aminosäure-Analyse bestimmte Pufferlösung aufgenommen und mit Hilfe eines konventionellen Aminosäure-Analysators quantitativ bestimmt. Im gleicher Weise wird auch Desulfatohirudin der Formel I analysiert. (Das Resultat kann auch als Mol-Verhältnis der Aminosäuren, insbesondere des Tyrosin-O-sulfats bzw. freien Tyrosins, zu Hirudin ausgedrückt werden) (Für die Kontrollproben werden sowohl Hirudin als auch CPY jeweils allein derselben Prozedur unterzogen.)

Auf gleiche Weise werden Proben von je 25 µl aus dem Desulfatierungs-Vorversuch nach Zerstörung der ARS-Aktivität durch kurzfristiges Erhitzen jeweils mit 2 µg CPY (in Form der Lösung Ba) versetzt und 30 min bei 25°C inkubiert. Nach Zugabe von 5 µl Trifluoressigsäure, Zentrifugation und Lyophilisation des

Überstandes werden freigesetzte Aminosäuren im Analysator quantitativ bestimmt. — (Kontrollansätze werden einzeln gefahren mit: (1) Hirudin, (2) CPY, (3) ARS, (4) Hirudin + CPY, (5) Hirudin + ARS, (6) CPY + ARS.)

Beispiel 2

Material

Hirudin: 1,5 Mg Hirudin (nachgereinigt mittels HPLC).

Arylsulfatase (aus Helix pomatia): Suspension der Fa. Boehringer: 5 mg/ml = 5 E/mg. Die Arylsulfatase (ARS) wird vor dem Versuch an einer PDS 10-Säule entsalzt. 100 µl Suspension werden ad 2,5 ml Puffer (0,1 M $NH_4Ac$, pH 5,5) aufgefüllt, auf die PD 10-Säule gegeben und mit 3 ml Puffer eluiert. Die Extinktion der eluierten Lösung beträgt $E_{280}$ = 0,139, 100 µl davon entsprachen 16 µg ARS.

Methode

Hirudin wird zu 2 µg/µl Puffer (0, 1 M $NH_4Ac$, pH 5,5) gelöst. Zu 20 µg Hirudin (≙ 10 µl) werden 100 µl der ARS-Lösung in Puffer gegeben.

Gewichtsverhältnis von Enzym (ARS): Substrat (Hirudin) = 1:1,25.

Man führt einen Ansatz in präparativen Maßstab 22 h bei 25°C durch. Dabei wird die Umsetzung mittels HPLC-Analyse verfolgt, wobei pro Bestimmung 2,7 µg Inhibitor eingesetzt werden.

Es ergibt sich, daß das Hirudin nach 6 h zu 90% zu Desulfatohirudin der Formel (I) desulatisiert ist. Nach 22 h liegt Desulfatohirudin der Formel (I) im Gemisch mit Desulfatohirudin der Formel (II) vor (I:II = 55:45). Dieses Gemisch kann mittels der in Beispiel 1 angegebenen Chromatographie-Verfahren getrennt werden.

Die Protein-chemische Charakterisierung der Endprodukte erfolgt mit der Dansylchlorid-Methode (N-terminale Bestimmung), durch Abbau mit Carboxypeptidase Y (C-terminale Bestimmung) und durch 24- bzw. 48stündige Aminosäure-Analyse.

**Patentansprüche für die Vertragsstaaten: BE CH DE FR GB IT LI LU NL SE**

1. Desulfatohirudine der Formel I

```
                                            10
H-Val-Val-Tyr-Thr-Asp-Cys-Thr-Glu-Ser-Gly-
                                            20
 -Gln-Asn-Leu-Cys-Leu-Cys-Glu-Gly-Ser-Asn-
                                            30
 -Val-Cys-Gly-Gln-Gly-Asn-Lys-Cys-Ile-Leu-   (I)
                                            40
 -Gly-Ser-Asp-Gly-Glu-Lys-Asn-Gln-Cys-Val-
                                            50
 -Thr-Gly-Glu-Gly-Thr-Pro-Lys-Pro-Gln-Ser-
                                            60
 -His-Asn-Asp-Gly-Asp-Phe-Glu-Glu-Ile-Pro-
 -Glu-Glu-Tyr-Leu-Gln-OH
```

und der Formel II

```
                                   10
H-Val-Val-Tyr-Thr-Asp-Cys-Thr-Glu-Ser-Gly-
                                   20
 -Gln-Asn-Leu-Cys-Leu-Cys-Glu-Gly-Ser-Asn-
                                   30
 -Val-Cys-Gly-Gln-Gly-Asn-Lys-Cys-Ile-Leu-   (II)
                                   40
 -Gly-Ser-Asp-Gly-Glu-Lys-Asn-Gln-Cys-Val-
                                   50
 -Thr-Gly-Glu-Gly-Thr-Pro-Lys-Pro-Gln-Ser-
                                   60
 -His-Asn-Asp-Gly-Asp-Phe-Glu-Glu-Ile-Pro-
 -Glu-Glu-Tyr-OH
```

worin die Reste -Cys- paarweise in gleicher Anordnung wie im Hirudin durch Disulfid-Brücken verknüpft sind, und deren Salze.

2. Desulfatohirudine und deren Salze nachAnspruch 1, erhältlich durch hydrolytische Desulfatierung von Hirudin.

3. Verfahren zur Herstellung der Desulfatohirudine und deren Salzen nach Anspruch 1, dadurch gekennzeichnet, daß man im Hirudin die Sulfatmonoestergruppe vom phenolischen Hydroxyl des Tyrosin-Restes in Stellung 63 hydrolytisch abspaltet und gegebenenfalls den C-terminalen Dipeptidylrest Leu-Gln abspaltet.

4. Verfahren gemäß Anspruch, dadurch gekennzeichnet, daß man die hydrolytische Abspaltung mit einer Arylsulfatase durchführt.

5. Verfahren gemäß Anspruch 3, dadurch gekennzeichnet, daß man die hydrolytische Abspaltung unter saurer Katalyse durchführt.

6. Pharmazeutische Mittel, enthaltend wenigstens eines der Desulfatohirudine oder deren pharmakologisch verträgliche Salze gemäß Anspruch 1, gegebenenfalls zusammen mit mindestens einem pharmazeutischen Träger oder Hilfsmaterial.

7. Verwendung der Desulfatohirudine nach Anspruch 1 oder der pharmakologisch verträglichen Salze davon zur Herstellung eines pharmazeutischen Mittels zur Hemmung der Gerinnung des Blutes von Menschen und Säugetieren.

8. Verwendung der Desulfatohirudine nach Anspruch 1 oder deren Salze als Reagens zur analytischen Bestimmung von Thrombin.

**Patentansprüche für den Vertragsstaat: AT**

1. Verfahren zur Herstellung der Desulfatohirudine der Formel I

```
                                             10
H-Val-Val-Tyr-Thr-Asp-Cys-Thr-Glu-Ser-Gly-
                                             20
 -Gln-Asn-Leu-Cys-Leu-Cys-Glu-Gly-Ser-Asn-
                                             30
 -Val-Cys-Gly-Gln-Gly-Asn-Lys-Cys-Ile-Leu-   (I)
                                             40
 -Gly-Ser-Asp-Gly-Glu-Lys-Asn-Gln-Cys-Val-
                                             50
 -Thr-Gly-Glu-Gly-Thr-Pro-Lys-Pro-Gln-Ser-
                                             60
 -His-Asn-Asp-Gly-Asp-Phe-Glu-Glu-Ile-Pro-
 -Glu-Glu-Tyr-Leu-Gln-OH
```

und der Formel II

```
                                             10
H-Val-Val-Tyr-Thr-Asp-Cys-Thr-Glu-Ser-Gly-
                                             20
 -Gln-Asn-Leu-Cys-Leu-Cys-Glu-Gly-Ser-Asn-
                                             30
 -Val-Cys-Gly-Gln-Gly-Asn-Lys-Cys-Ile-Leu-   (II)
                                             40
 -Gly-Ser-Asp-Gly-Glu-Lys-Asn-Gln-Cys-Val-
                                             50
 -Thr-Gly-Glu-Gly-Thr-Pro-Lys-Pro-Gln-Ser-
                                             60
 -His-Asn-Asp-Gly-Asp-Phe-Glu-Glu-Ile-Pro-
 -Glu-Glu-Tyr-OH
```

worin die Reste -Cys- paarweise in gleicher Anordnung wie im Hirudin durch Disulfid-Brücken verknüpft sind, und der Salze davon, dadurch gekennzeichnet, daß man im Hirudin die Sulfatmonoestergruppe vom phenolischen Hydroxyl des Tyrosin-Restes in Stellung 63 hydrolytisch abspaltet und gegebenenfalls den C-terminalen Dipeptidylrest Leu-Gln abspaltet.

2. Verfahren gemäß Anspruch 1, dadurch gekennzeichnet, daß man die hydrolytische Abspaltung mit einer Arylsulfatase durchführt.

3. Verfahren gemäß Anspruch 1, dadurch gekennzeichnet, daß man die hydrolytische Abspaltung unter saurer Katalyse durchführt.

4. Verfahren zur Herstellung eines pharmazeutischen Mittels, dadurch gekennziechnet, daß man mindestens eines der Desulfatohirudine oder ein pharmakologisch verträgliches Salz davon gemäß Anspruch 1 in einer zur Verabreichung geeigeneten Form zur Verfügung stellt und gegebenenfalls mindstens einem pharmazeutischen Träger oder Hilfsmaterial einverleibt.

5. Verwendung der Desulfatohirudine nach Anspruch 1 oder der pharmakologisch verträglichen Salze davon zur Herstellung eines pharmazeutischen Mittels zur Hemmung der Gerinnung des Blutes von Menschen und Säugetieren.

6. Verwendung der Desulfatohirudine nach Anspruch 1 oder deren Salze als Reagens zur analytischen Bestimmung von Thrombin.

**Revendications pour les Etats contractants: BE CH DE FR GB IT LI LU NL SE**

1. Désulfatohirudines de formule I

```
                                             10
H-Val-Val-Tyr-Thr-Asp-Cys-Thr-Glu-Ser-Gly-
                                             20
 -Gln-Asn-Leu-Cys-Leu-Cys-Glu-Gly-Ser-Asn-
                                             30
 -Val-Cys-Gly-Gln-Gly-Asn-Lys-Cys-Ile-Leu-   (I)
                                             40
 -Gly-Ser-Asp-Gly-Glu-Lys-Asn-Gln-Cys-Val-
                                             50
 -Thr-Gly-Glu-Gly-Thr-Pro-Lys-Pro-Gln-Ser-
                                             60
 -His-Asn-Asp-Gly-Asp-Phe-Glu-Glu-Ile-Pro-
 -Glu-Glu-Tyr-Leu-Gln-OH
```

et de formule II

```
                                             10
H-Val-Val-Tyr-Thr-Asp-Cys-Thr-Glu-Ser-Gly-
                                             20
 -Gln-Asn-Leu-Cys-Leu-Cys-Glu-Gly-Ser-Asn-
                                             30
 -Val-Cys-Gly-Gln-Gly-Asn-Lys-Cys-Ile-Leu-   (II)
                                             40
 -Gly-Ser-Asp-Gly-Glu-Lys-Asn-Gln-Cys-Val-
                                             50
 -Thr-Gly-Glu-Gly-Thr-Pro-Lys-Pro-Gln-Ser-
                                             60
 -His-Asn-Asp-Gly-Asp-Phe-Glu-Glu-Ile-Pro-
 -Glu-Glu-Tyr-OH
```

dans lesquelles les motifs -Cys- sont reliés par paires par des ponts disulfure, dans la même disposition que dans l'hirudine, et leurs sels.

2. Désulfatohirudines et leurs sels selon la revendication 1, obtenues par désulfatation de l'hirudine par hydrolyse.

3. Procédé de préparation des désulfatohirudines et de leurs sels selon la revendication 1, caractérisé en ce que, partant de l'hirudine, on scinde par hydrolyse le groupe monoester sulfurique du groupe hydroxy phénolique du motif de tyrosine en position 63 et éventuellemente on scinde Leu-Gln sur le C-terminale.

4. Procédé selon la revendication 3, caractérisé en ce que la scission par hydrolyse et réalisée à l'aide d'une arylsulfatase.

5. Procédé selon la revendication 3, caractérisé en ce que la scission par hydrolyse est réalisée avec catalyse acide.

6. Produit pharmaceutique contenant au moins une des désulfatohirudines ou de leurs sels acceptables pour l'usage pharmaceutique selon la revendication 1, éventuellement avec au moins un véhicule ou produit auxiliaire pharmaceutique.

7. Utilisation des désulfatohirudines selon la revendication 1 ou de leurs sels acceptables pour l'usage pharmaceutique, par la préparation d'un produit pharmaceutique pour l'inhibition de la coagulation du sang des humains et des mammifères.

8. Utilisation des désulfatohirudines selon la revendication 1 ou de leurs sels en tant que réactifs pour la détermination analytique de la thrombine.

**Revendications pour l'Etat contractant: AT**

1. Procédé de préparation des désulfatohirudines de formule I

```
                                          10
H-Val-Val-Tyr-Thr-Asp-Cys-Thr-Glu-Ser-Gly-
                                          20
 -Gln-Asn-Leu-Cys-Leu-Cys-Glu-Gly-Ser-Asn-
                                          30
 -Val-Cys-Gly-Gln-Gly-Asn-Lys-Cys-Ile-Leu-   (I)
                                          40
 -Gly-Ser-Asp-Gly-Glu-Lys-Asn-Gln-Cys-Val-
                                          50
 -Thr-Gly-Glu-Gly-Thr-Pro-Lys-Pro-Gln-Ser-
                                          60
 -His-Asn-Asp-Gly-Asp-Phe-Glu-Glu-Ile-Pro-
 -Glu-Glu-Tyr-Leu-Gln-OH
```

et de formule II

```
                                          10
H-Val-Val-Tyr-Thr-Asp-Cys-Thr-Glu-Ser-Gly-
                                          20
 -Gln-Asn-Leu-Cys-Leu-Cys-Glu-Gly-Ser-Asn-
                                          30
 -Val-Cys-Gly-Gln-Gly-Asn-Lys-Cys-Ile-Leu-   (II)
                                          40
 -Gly-Ser-Asp-Gly-Glu-Lys-Asn-Gln-Cys-Val-
                                          50
 -Thr-Gly-Glu-Gly-Thr-Pro-Lys-Pro-Gln-Ser-
                                          60
 -His-Asn-Asp-Gly-Asp-Phe-Glu-Glu-Ile-Pro-
 -Glu-Glu-Tyr-OH
```

dans lesquelles les motifs -Cys- sont reliés par paires par des ponts disulfure, dans la même disposition que dans l'hirudine, et de leurs sels caractérisé en ce que, partant de l'hirudine, on scinde par hydrolyse le groupe monoester sulfurique du groupe hydroxy phénolique du motif de tyrosine en position 63 et éventuellement on scinde Leu-Gln sur le C-terminale.

2. Procédé selon la revendication 1, caractérisé en ce que la scission par hydrolyse et réalisée à l'aide d'une arylsulfatase.

3. Procédé selon la revendication 1, caractérisé en ce que la scission par hydrolyse est réalisée avec catalyse acide.

4. Procédé de préparation d'un produit pharmaceutique, caractérisé en ce que l'on met au moins une des désulfatohirudines ou de leurs sels acceptables pour l'usage pharmaceutique, selon la revendication 1, sous une forme appropriée à l'administration et, le cas échéant, on incorpore au moins un véhicule ou produit auxiliaire pharmaceutique.

5. Utilisation des désulfatohirudines selon la revendication 1 ou de leurs sels acceptables pour l'usage pharmaceutique, par la préparation d'un produit pharmaceutique pour l'inhibition de la coagulation du sang des humains et des mammifères.

6. Utilisation des désulfatohirudines selon la revendication 1 ou de leurs sels en tant que réactifs pour la détermination analytique de la thrombine.

**Claims for the Contracting States: BE CH DE FR GB IT LI LU NL SE**

1. A desulfatohirudin of the formula I

```
                                            10
H-Val-Val-Tyr-Thr-Asp-Cys-Thr-Glu-Ser-Gly-
                                            20
 -Gln-Asn-Leu-Cys-Leu-Cys-Glu-Gly-Ser-Asn-
                                            30
 -Val-Cys-Gly-Gln-Gly-Asn-Lys-Cys-Ile-Leu-   (I)
                                            40
 -Gly-Ser-Asp-Gly-Glu-Lys-Asn-Gln-Cys-Val-
                                            50
 -Thr-Gly-Glu-Gly-Thr-Pro-Lys-Pro-Gln-Ser-
                                            60
 -His-Asn-Asp-Gly-Asp-Phe-Glu-Glu-Ile-Pro-
 -Glu-Glu-Tyr-Leu-Gln-OH
```

or of the formula II

```
                                            10
H-Val-Val-Tyr-Thr-Asp-Cys-Thr-Glu-Ser-Gly-
                                            20
 -Gln-Asn-Leu-Cys-Leu-Cys-Glu-Gly-Ser-Asn-
                                            30
 -Val-Cys-Gly-Gln-Gly-Asn-Lys-Cys-Ile-Leu-   (II)
                                            40
 -Gly-Ser-Asp-Gly-Glu-Lys-Asn-Gln-Cys-Val-
                                            50
 -Thr-Gly-Glu-Gly-Thr-Pro-Lys-Pro-Gln-Ser-
                                            60
 -His-Asn-Asp-Gly-Asp-Phe-Glu-Glu-Ile-Pro-
 -Glu-Glu-Tyr-OH
```

wherein the -Cys- residues are linked in pairs by disulfide bridges in the same manner as in hirudin, or a salt thereof.

2. A desulfatohirudin or a salt thereof according to claim 1, obtainable by hydrolytic desulfation of hirudin.

3. A process for the preparation of a desulfatohirudin or a salt thereof according to claim 1, which comprises removing by hydrolysis, in hirudin, the sulfate monoester group from the phenolic hydroxyl group of the tyrosine residue in position 63 and optionally removing the C-terminal dipeptidyl radical Leu-Gln.

4. A process according to claim 3, wherein the hydrolytic removal is carried out with an arylsulfatase.

5. A process according to claim 3, wherein the hydrolytic removal is carried out under the conditions of acid catalysis.

6. A pharmaceutical composition containing at least one desulfatohirudin or a pharmacologically acceptable salt thereof according to claim 1, optionally together with at least one pharmaceutical carrier or excipient.

7. The use of a desulfatohirudin according to claim 1 or a pharmacologically acceptable salt thereof for the preparation of a pharmaceutical composition for inhibiting the clotting of blood in human and mammals.

8. The use of a desulfatohirudin according to claim 1 or a salt thereof as a reagent for the analytical determination of thrombin.

**Claims for Contracting State: AT**

1. A process for the preparation of a desulfatohirudin of the formula I

```
                                         10
H-Val-Val-Tyr-Thr-Asp-Cys-Thr-Glu-Ser-Gly-
                                         20
 -Gln-Asn-Leu-Cys-Leu-Cys-Glu-Gly-Ser-Asn-
                                         30
 -Val-Cys-Gly-Gln-Gly-Asn-Lys-Cys-Ile-Leu-   (I)
                                         40
 -Gly-Ser-Asp-Gly-Glu-Lys-Asn-Gln-Cys-Val-
                                         50
 -Thr-Gly-Glu-Gly-Thr-Pro-Lys-Pro-Gln-Ser-
                                         60
 -His-Asn-Asp-Gly-Asp-Phe-Glu-Glu-Ile-Pro-
 -Glu-Glu-Tyr-Leu-Gln-OH
```

or of the formula II

```
                                         10
H-Val-Val-Tyr-Thr-Asp-Cys-Thr-Glu-Ser-Gly-
                                         20
 -Gln-Asn-Leu-Cys-Leu-Cys-Glu-Gly-Ser-Asn-
                                         30
 -Val-Cys-Gly-Gln-Gly-Asn-Lys-Cys-Ile-Leu-   (II)
                                         40
 -Gly-Ser-Asp-Gly-Glu-Lys-Asn-Gln-Cys-Val-
                                         50
 -Thr-Gly-Glu-Gly-Thr-Pro-Lys-Pro-Gln-Ser-
                                         60
 -His-Asn-Asp-Gly-Asp-Phe-Glu-Glu-Ile-Pro-
 -Glu-Glu-Tyr-OH
```

wherein the -Cys- residues are linked in pairs by disulfide bridges in the same manner as in hirudin, or a salt thereof, which process comprises removing by hydrolysis, in hirudin, the sulfate monoester group from the phenolic hydroxyl group of the tyrosine residue in position 63 and optionally removing the C-terminal dipeptidyl radical Leu-Gln.

2. A process according to claim 1, wherein the hydrolytic removal is carried out with an arylsulfatase.

3. A process according to claim 1, wherein the hydrolytic removal is carried out under the conditions of acid catalysis.

4. A process for the preparation of a pharmaceutical composition, wherein at least one desulfatohirudin or a pharmacologically acceptable salt thereof according to claim 1 is prepared in the form suitable for administration and optionally incorporated into at least one pharmaceutical carrier or excipient.

5. The use of a desulfatohirudin according to claim 1 or a pharmacologically acceptable salt thereof for the preparation of a pharmaceutical composition for inhibiting the clotting of blood in human and mammals.

6. The use of a desulfatohirudin according to claim 1 or a salt thereof as a reagent for the analytical determination of thrombin.